# EUROPÄISCHE PATENTSCHRIFT

(11) **EP 1 361 825 B1**
(45) Veröffentlichungstag und Bekanntmachung des Hinweises auf die Patenterteilung: **29.11.2006**
(21) Anmeldenummer: 02716608.1
(22) Anmeldetag: 21.02.2002
(51) Int. Cl.: A61B 6/14

(54) **ANORDNUNG UND VERFAHREN ZUR POSITIONIERUNG EINES DENTALEN DIGITALEN RÖNTGENGERÄTES**
ARRANGEMENT AND METHOD FOR POSITIONING A DENTAL DIGITAL X-RAY MACHINE
PROCEDE ET SYSTEME POUR POSITIONNER UN APPAREIL DE RADIOGRAPHIE NUMERIQUE DENTAIRE

(30) Priorität: 21.02.2001 DE 10108298
(43) Veröffentlichungstag der Anmeldung: 19.11.2003
(73) Patentinhaber: Sirona Dental Systems GmbH, 64625 Bensheim (DE)
(72) Erfinder: DALPIAZ, Michael, 64673 Zwingenberg (DE); SCHULZE-GANZLIN, Ulrich, 64653 Lorsch (DE); GÜNTHER, Werner, 64625 Bensheim (DE); ZIMMERMANN, Jürgen, 64584 Biebesheim (DE)
(74) Vertreter: Sommer, Peter
(86) Internationale Anmeldenummer: PCT/DE2002/000636
(87) Internationale Veröffentlichungsnummer: WO 2002/065918

(56) Entgegenhaltungen:
- EP-A- 0 858 773
- US-A- 4 813 060
- STELT VAN DER P F: "THE E-MAGO IMAGE PROCESSING ENVIRONMENT FOR DIRECT DIGITAL RADIOGRAPHY IN DENTISTRY" BIOMECHANICS, REHABILITATION, ELECTRICAL PHENOMENA, BIOMATERIALS. SAN DIEGO, OCT. 28 - 31, 1993, PROCEEDINGS OF THE ANNUAL INTERNATIONAL CONFERENCE OF THE ENGINEERING IN MEDICINE AND BIOLOGY SOCIETY, NEW YORK, IEEE, US, Bd. 3 CONF. 15, 28. Oktober 1993 (1993-10-28), Seiten 1623-1624, XP000453000

## Beschreibung

### Technisches Gebiet

Die Erfindung betrifft eine Anordnung und ein Verfahren zur Positionierung eines digitalen dentalen Röntgengerätes. Im dentalmedizinischen Bereich besteht ein hoher Zeitaufwand für die Positionierung von Röntgengeräten. Dabei werden vom Arzt fest vorgegebenen Arbeitsfolgen eingehalten. In einer möglichen Arbeitsfolge wird ausgehend von einer Großaufnahme (Z.B. Panoramaübersichtsaufnahme) zu kleineren Aufnahmen übergegangen (z.B. Intraoral-Detail-Aufnahme oder andere Schichtaufnahme von Details, z.B. von einzelnen Zähnen). Die Bestimmung des Detailbereichs erfolgt auf der Grundlage der Übersichtsaufnahme (Panorama-Aufnahme).

### Stand der Technik

Ein Röntgengerät zur Erstellung von Panorama-Schichtaufnahmen und Einzelaufnahmen hiervon ist aus der DE 35 45 509 (US 4,847,881) und der DE 35 45 493 (US 4,813,060) bekannt. Digitale Röntgenaufnahmen für Panoramaschichtaufnahmen und cephalometrische Aufnahmen sind aus der EP 0 632 994 (US 5,511,106) und aus des EP 0 858 773 bekannt. Die Erstellung digitaler Intraoral-Aufnahmen mit einem Intraoralsensor sind aus der EP 0 643 901 (US 5,513,252) bekannt.

Die Anweisungen für die weitere Untersuchung werden in der Regel mündlich oder schriftlich weitergegeben.

Aus der DE 197 03 556 A1 ist ein Verfahren zur Positionsbestimmung bei der Röntgenbildgebung bekannt, bei dem mit einem Röntgengerät mindestens ein Röntgenbild eines Untersuchungsobjekts erstellt wird. An dem Röntgengerät ist eine Detektoranordnung vorgesehen, mittels welcher die Position einer am Untersuchungsobjekt oder relativ zum Untersuchungsobjekt ortsfest angeordnet Markeranordnung in einem mit der Detektoranordnung verkoppelten Detektorkoordinatensystem erfasst wird. Die Position des Röntgengeräts wird in einem Objektkoordinatensystem bestimmt, welches mit dem Untersuchungsobjekt verkoppelt ist. Anschließend wird die Position eines als Bildpunkt in einem Röntgenbild abgebildeten Objektpunkts im Objektkoordinatensystem bestimmt. Mittels dieses Verfahrens ist die exakte Zuordnung eines Punktes in einem Röntgenbild zu einem Punkt in oder am Untersuchungsobjekt möglich. Darüber hinaus ist auch die exakte Positionsbestimmung eines Behandlungsinstrumentes möglich.

Aufgabe der Erfindung ist es, eine Anordnung und ein Verfahren bereitzustellen, die es ermöglichen in effizienter Weise Detailaufnahmen durchzuführen.

### Darstellung der Erfindung

Diese Aufgabe wird durch eine Anordnung und ein Verfahren mit den Merkmalen der unabhängigen Ansprüche gelöst.

Insbesondere durch eine Anordnung zur Positionierung eines Röntgengerätes, die Ein- und Ausgabegeräte zur interaktiven Steuerung aufweist. Durch die Ein- und Ausgabegeräte erhält der Benutzer die Möglichkeit, die Anordnung zu steuern. Bei diesen Geräten handelt es sich vorzugsweise um eine Tastatur und einen Monitor, die in Verbindung mit einem Zeigegerät zur Bestimmung von Bereichen verwendet werden. In einem Speicherbereich ist mindestens eine digitalisierte, vorzugsweise individuelle Röntgenaufnahme und Röntgengerätinformationen abgelegt. Die Röntgengerätinformationen werden in Relation zu der Röntgenaufnahme gespeichert. Hierdurch ist es möglich, dass Bereiche einer Röntgenaufnahme bestimmten Röntgengerätinformationen zugeordnet werden können. Bei diesen Röntgengerätinformationen handelt es sich vorzugsweise um Positionsparameter der beweglichen Teile. Somit können zu einem bestimmten Bereich einer Röntgenaufnahme die entsprechenden Parameter des Röntgengeräts bestimmt werden. Umgekehrt kann das Röntgengerät durch die Röntgenaufnahme gesteuert werden.

Die Anordnung weist weiterhin eine Schnittstelle auf, über die Informationen mit dem Röntgengeräte ausgetauscht werden. Zur Bestimmung des Bereichs, der in einer weiteren Detailaufnahmen berücksichtigt werden soll, sind Mittel vorhanden, um Bereiche auf der digitalisierten Röntgenaufnahme auszuwählen. Bei diesen Mitteln handelt es sich vorzugsweise um ein Zeigegerät, mit dem ein bereits bestehender Bereich ausgewählt werden kann oder durch den ein noch nicht vorhandener Bereich bestimmt werden kann. Dieses Zeigegerät kann z. B. eine Maus sein, mit der ein Bereich bestimmt wird oder eingezeichnet wird.

Ein weiterer Bestandteil der vorliegenden Erfindung ist eine Bearbeitungseinheit, die auf der Grundlage der digitalisierten, vorzugsweise individuellen Röntgenaufnahme, den dazu vorliegenden Röntgengerätinformationen und des ausgewählten Bereichs Berechnungen durchführt, um Steuerungsdaten zu ermitteln, die das Röntgengerät so steuern, dass es den ausgewählten Bereich abdeckt. Diese Berechnungen basieren vorzugsweise auf den Bahninformationen, die, wie bereits oben beschrieben wurde, den Aufnahmeinformationen zugeordnet sind. Die Bahninformationen geben Aufschluss darüber, welche Bewegung das Röntgengerät zu einem bestimmten Zeitpunkt durchgeführt hat. Es handelt sich somit um Koordinaten des Röntgengerätes, die in Relationen zu einem bestimmten Zeitpunkt stehen.

Aus Strom- und Spannungsparametern kann die Intensität der Strahlung sowohl auf der Seite der Strahlungsquelle als auch auf der Seite des Sensors ermittelt werden. Hieraus lassen sich Rückschlüsse auf die Bildinformationen treffen.

Die Abbildung von Spannungswerten auf Grauwerte, in denen die Röntgenaufnahme dargestellt wird, neigt dazu fehlerträchtig zu sein. Um diese Fehlerquelle zu umgehen, ist es notwendig, Kenntnisse über die Abbildung zu besitzen. Hierdurch können Abweichungen rechnerisch eliminiert werden.

In einer bevorzugten Ausführungsform fließt bei der Bestimmung der Steuerungsdaten die Untersuchungsart und das Diagnoseziel entsprechend der klinischen Fragestellung in die Berechung ein. Beide zusammen geben die Aufnahmeart vor. So sind die Parameter des Röntgengerätes unterschiedlich zu setzen, wenn eine Untersuchung der Wurzel erfolgen soll oder wenn eine Untersuchung des Kariesbefalls vorgenommen werden soll, da letzterer vornehmlich auf der Oberfläche des Zahnes angesiedelt ist.

Auf der Basis von Patientendaten, wie Größe, Gewicht, Typ, Rasse, Alter, Kieferform und bisherigen Behandlungen können ebenfalls Aussagen über die Steuerungsdaten für das Röntgengerät getroffen werden. So ist die Strahlung geringer zu wählen, bei einem kleinen Kindergebiss.

Um die Benutzung möglichst einfach zu gestalten, können die Zähnen bereits durch Mustererkennungsalgorithmen im Vorfeld erkannt werden, sodass der Benutzer nur noch die Zähnen auswählen muss, für die er weitere Aufnahmen plant. Der Benutzer muss somit nicht einen Bereich manuell auf der Abbildung einzeichnen.

Auf der Basis von statistischen und stochastischen Daten können weitere Aussagen bezüglich der Anatomie des Patienten getroffen werden, die dann wiederum in die Berechnung der Steuerungsdaten einfließen können.

Eih weiterer Gegenstand der vorliegenden Erfindung ist ein Verfahren zur Positionierung eines Röntgengerätes. In einem ersten Schritt wird mindestens eine digitalisierte, vorzugsweise individuelle Röntgenaufnahme geladen und dargestellt. Die Darstellung erfolgten in der Regel auf einer Anzeigevorrichtung, insbesondere einem Bildschirm. In einem zweiten Schritt werden die Koordinaten von Bereichen bestimmt, die in einer weiteren Röntgenaufnahme abgebildet werden sollen. Diese Bestimmung kann entweder manuell durch Einzeichnen der Bereiche erfolgen, oder es werden bereits Vorschläge unterbreitet, die der Benutzer interaktiv auswählen kann. In einem dritten Schritt werden Röntgengerätinformationen geladen, aus denen entnommen werden kann, welche Parameter benötigt werden, um den ausgewählten Bereich in einer nächsten Aufnahme abzubilden. In einem vierten Schritt werden auf der Grundlage der digitalisierten Röntgenaufnahme, den dazu vorliegenden Röntgengerätinformationen und des ausgewählten Bereichs Berechnungen durchführt, um Steuerungsdaten zu ermitteln, die das Röntgengerät so steuern, dass der ausgewählte Bereich abgebildet wird. Die einzelnen Berechnungsschritte wurden bereits oben beschrieben.

Bei den Informationen des Röntgengerätes handelt es sich vorzugsweise um Koordinaten der Bahnkurve, die in Relation zu der digitalisierten Röntgenaufnahme abgelegt sind. Mit Hilfe dieser Informationen kann für den ausgewählten Bereich ein Segment der Bahnkurve berechnet werden.

In die Berechnung können ebenfalls Strom- und Spannungsparameter einfließen, die in Relation zu der digitalisierten Röntgenaufnahme abgelegt sind.

Darüber hinaus können auch die Betriebsparameter des Sensors variiert werden, beispielsweise die Austaktfrequenz, sowie die Parameter zur Berechnung des Röntgenbildes anhand der vom Sensor erhaltenen Signale, beispielsweise bei dem Auslesen von Vollbildern die in der Bearbeitungseinheit stattfindende Überlagerung zur nachträglichen Festlegung der scharfen Schicht.

Neben den Patientendaten, wie Größe, Gewicht, Typ, Rasse, Alter, Kieferform und bisherige Behandlungen können bei der Bestimmung der Steuerungsdaten die Untersuchungsart und das Diagnoseziel entsprechend der klinischen Fragestellung in die Berechung einfließen.

Eine automatische Erkennung der Bereiche, insbesondere der Zähne, wird durch Mustererkennungsalgorithmen ermöglicht. Diese Bereiche können jedoch auch manuell verändert oder bestimmt werden.

Wie bei der Anordnung werden statistische und stochastische Verknüpfungen der einzelnen Parameter vorgenommen.

### Kurzbeschreibung der Zeichnung

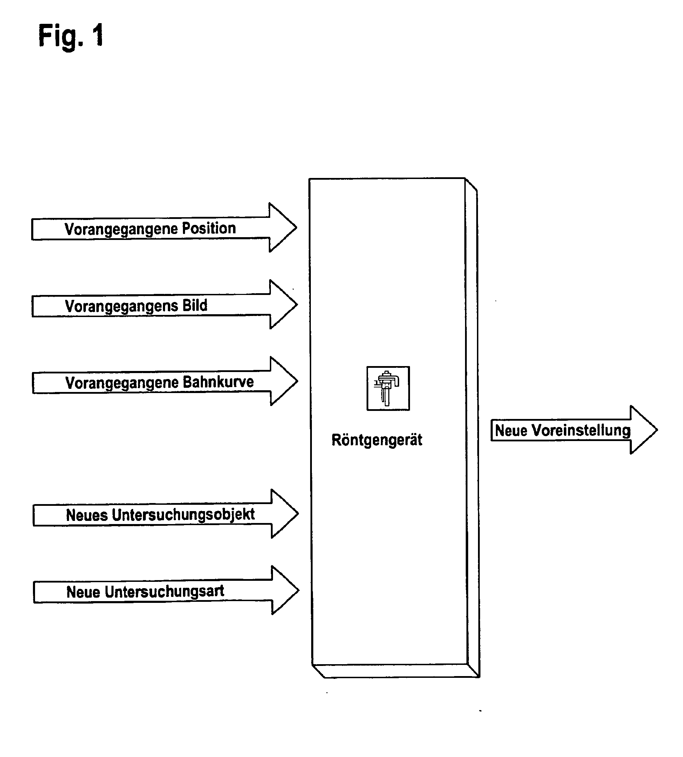

Im folgenden wird die Erfindung anhand von Ausführungsbeispielen näher erläutert, die in der Figur schematisch dargestellt sind. Es zeigt die einzige
- Fig. 1: den schematischer Ablauf bei der Bestimmung der Voreinstellung des Röntgengerätes,
- Fig. 2: die automatische Auswahl eines zu untersuchenden Bereichs anhand einer digitalisierten dentalen Panorama-Schichtaufnahme und die
- Fig. 3: die manuelle Auswahl eines zu untersuchenden Bereichs anhand einer digitalisierten dentalen Panorama-Schichtaufnahme.

### Ausführungsbeispiel

In die Berechnung der Voreinstellung fließen die vorangegangene Position, das vorangegangene Bild und die vorangegangene Bahnkurve. Weiterhin wird das neue Untersuchungsobjekten für die Berechnung benötigt und die neue Untersuchungsart. Die neuen Einstellungen werden in der bereits beschriebenen Form berechnet. Die Einstellung des Röntgengerät wird auf der Grundlage der so ermittelten Parameter durchgeführt.

Zusätzlich zu der Positionierung eines Strahlers des Röntgengeräts können eine Aufbissvorrichtung, eine Kinn- und/oder Stirnfixierung und/oder ein Ohrhalter ausgerichtet werden, um den Patienten in eine zur Erstellung der Aufnahme geeignete Lage zu bringen. Dadurch wird die Reproduzierbarkeit für eine spätere Röntgenaufnahme zum Vergleich oder zur Feststellung des Verfahrensverlaufs gewährleistet. Die Positionierung von Röntgengerät und/oder Patient bezieht sich auch auf mit dem Röntgengerät verbundene Strahlungsempfänger zur Erstellung von Panorama-Schichtaufnahmen (PAN) oder Transversalschichtaufnahmen (TSA) als auch frei bewegliche Sensoren zur intraoralen Anordnung (IO-Sensoren) gegebenenfalls unter Verwendung von speziellen Haltern zur Lagefixierung im Mund des Patienten.

Als digitalisierte Röntgenaufnahme kann in einem einfachsten Fall der Ausführung ein standardisiertes Röntgenbild mit vorgegebenen Teilbereichen der Zähne verwendet werden, um zusammen mit den allgemeinen Gerätedaten eine Grobeinstellung des Röntgengeräts gemäß der getroffenen Aufnahmeauswahl vorzunehmen.

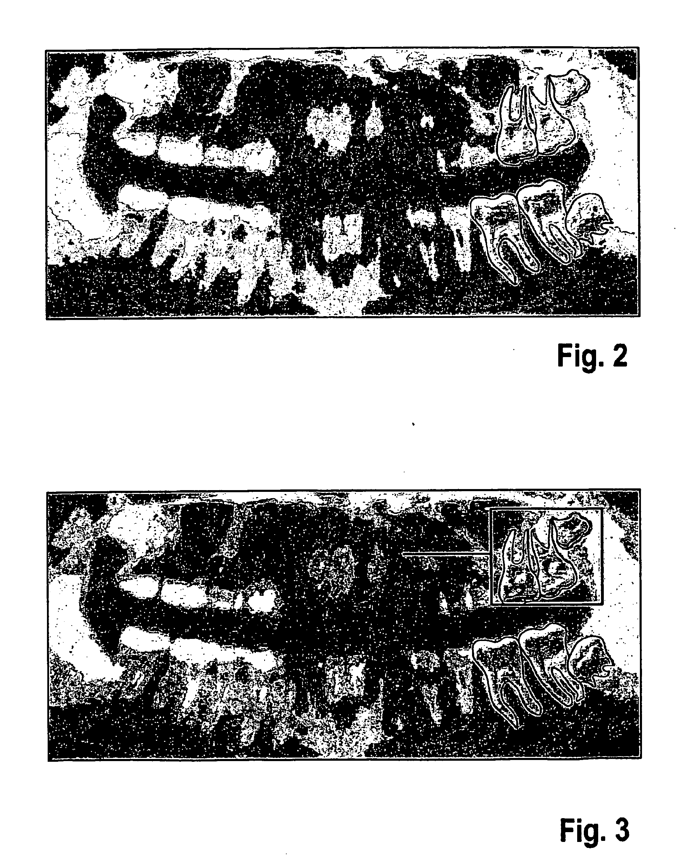

In den Fig. 2 und 3 sind Auswahlmöglichkeiten eines zu untersuchenden Bereichs anhand einer digitalisierten dentalen Panorama-Schichtaufnahme gezeigt. Im Fall der Fig. 2 erfolgt die Auswahl des Bereichs über die Eingabe der interessierenden Zähne oder über ein Anklicken der als Teilbereiche der Panorama-Schichtaufnahme abgespeicherten Zähne, welche daraufhin hervorgehoben dargestellt sind. In Fig. 3 erfolgt die Auswahl des Bereichs über die Positionierung eines Auswahlrahmens, der in seiner Form und Lage einem Intraoral-Sensor nachgebildet sein kann.

Besonders vorteilhaft ist allerdings die Verwendung von bereits vom Patienten vorliegenden individuellen Röntgenaufnahmen, in denen die zur Erstellung der Aufnahme tatsächlich verwendeten Gerätedaten angespeichert sind. Darüber hinaus kann ein zur Positionierung verwendetes, vom Patienten selbst stammendes Röntgenbild eine patientenbezogene Zuordnung von Teilbereichen aufweisen, sodass die Auswahl des Teilbereichs innerhalb der patientenbezogenen Aufnahme eine erhöhte Positioniergenauigkeit mit sich bringt.

Eventuell ist es dabei erforderlich, zusätzlich zu der automatisch vorgenommenen Positionierung des Geräts manuelle Korrekturen vorzunehmen. Diese Korrekturen können ebenfalls zusammen mit der Bildinformation in der Röntgenaufnahme abgespeichert werden und stehen für zukünftige Aufnahmen zur Verfügung.

Die Anordnung und das Verfahren können so ausgestaltet sein, dass darüber hinaus nicht nur Einzelaufnahmen von der ausgewählten Position erstellt werden, sondern dass eine Aufnahmeserie ausgehend von der ausgewählten Position unter weiterer Verstellung des Röntgengeräts erfolgt, beispielsweise zur Erstellung mehrerer Schichten eines interessierenden Zahns als Transversalschichtaufnahme. Die Auswahl der Aufnahmeart erfolgt vor der Berechnung der Steuerungsdaten über zusätzliche Mittel und wird bei der Berechnung der Steuerungsdaten berücksichtigt. Darüber hinaus lassen sich für den Fall, dass bewegliche Mittel zur Patientenpositionierung vorgesehen sind, auch deren Steuerungsdaten erfassen bzw. für die nachfolgende Aufnahme auch wieder vorgeben.

Das Verfahren kann in Form einer Software nach einem oder mehreren der nachstehenden Verfahrensansprüche niedergelegt sein. Ein Datenträger kann eine ablauffähige Datenstruktur, die auf einem Computer ein Verfahren nach einem oder mehreren der nachstehenden Verfahrensansprüche realisiert, enthalten.

## Patentansprüche

1. Anordnung zur Positionierung eines dentalen Röntgengerätes,
- mit einem Ein- und Ausgabegerät zur interaktiven Steuerung,
- mit einem Speicherbereich, in dem mindestens eine digitalisierte dentale Röntgenaufnahme und der digitalisierte dentale Röntgenaufnahme zuordenbare Röntgengerätinformationen abgelegt sind,
- mit einer Schnittstelle, über die Informationen mit dem Röntgengerät ausgetauscht werden,
- mit einer Bearbeitungseinheit, die auf der Grundlage eines ausgewählten Bereichs Berechnungen durchführt, um Steuerungsdaten für das dentale Röntgengerät zu ermitteln,
- wobei das dentale Röntgengerät anhand der Steuerungsdaten so steuerbar ist, dass der ausgewählte Bereich bei der Erstellung einer dentalen Röntgenaufnahme abgedeckt ist, **dadurch gekennzeichnet, dass** Mittel vorhanden sind, um Bereiche auf der digitalisierten dentalen Röntgenaufnahme auszuwählen, und dass die Bearbeitungseinheit zur Berechnung des Steuerungsdaten für das Röntgengerät diese auf der Grundlage des ausgewählten Bereichs der digitalisierten dentalen Röntgenaufnahme und den dazu vorliegenden Röntgengerätinformationen berechnet.

2. Anordnung nach dem vorhergehenden Anspruch, **dadurch gekennzeichnet, dass** die digitalisierte Röntgenaufnahme eine individuelle Aufnahme des Patienten ist.

3. Anordnung nach dem vorhergehenden Anspruch mit einem Röntgengerät, welches für mehrere Aufnahmearten geeignet ist, **dadurch gekennzeichnet, dass** Mittel zur Auswahl der Aufnahmeart vorgesehen sind.

4. Anordnung nach einem oder mehreren der vorhergehenden Ansprüche mit Mitteln zur Positionierung eines Patienten in bezug auf das Röntgengerät, **dadurch gekennzeichnet, dass** die Steuerungsdaten die Mittel zur Positionierung des Patienten steuern.

5. Anordnung nach einem oder mehreren der vorhergehenden Ansprüche, **dadurch gekennzeichnet, dass** die Informationen des Röntgengerätes Koordinaten der Bahnkurve sind, die in Relation zu der digitalisierten Röntgenaufnahme abgelegt sind.

6. Anordnung nach einem oder mehreren der vorhergehenden Ansprüche, **dadurch gekennzeichnet, dass** Strom- und/oder Spannungsarameter in Relation zu der digitalisierten Röntgenaufnahme abgelegt sind.

7. Anordnung nach einem oder mehreren der vorhergehenden Ansprüche, **dadurch gekennzeichnet, dass** Informationen über die Grauwerte der Darstellung der Abbildung in Relation zu der digitalen Röntgenaufnahme abgelegt sind.

8. Anordnung nach einem oder mehreren der vorhergehenden Ansprüche, **dadurch gekennzeichnet, dass** bei der Bestimmung der Steuerungsdaten die Aufnahmeart in die Berechung einfließt.

9. Anordnung nach einem oder mehreren der vorhergehenden Ansprüche, **dadurch gekennzeichnet, dass** bei der Bestimmung der Steuerungsdaten das Diagnoseziel in die Berechnung einfließt.

10. Anordnung nach einem oder mehreren der vorhergehenden Ansprüche, **dadurch gekennzeichnet, dass** Patientendaten, wie Größe, Gewicht, Typ, Rasse, Alter, Kieferform und/oder bisherige Behandlungen bei der Bestimmung der Steuerungsdaten in die Berechnung einfließen.

11. Anordnung nach einem oder mehreren der vorhergehenden Ansprüche, **gekennzeichnet durch** Mittel, die Bereiche, insbesondere Zähne, **durch** Mustererkennungsalgorithmen automatisch erkennen.

12. Anordnung nach einem oder mehreren der vorhergehenden Ansprüche, **dadurch gekennzeichnet, dass** die Auswahlmittel so ausgebildet sind, dass Bereiche manuell auswählbar sind.

13. Anordnung nach einem oder mehreren der vorhergehenden Ansprüche, **dadurch gekennzeichnet, dass** statistische und/oder stochastische Verknüpfungen der einzelnen Parameter vorgenommen werden.

14. Anordnung nach einem oder mehreren der vorhergehenden Ansprüche, **dadurch gekennzeichnet, dass** Mittel zur Erstellung von Aufnahmeserien mit unterschiedlicher Positionierung ausgehend von der ausgewählten Position vorgesehen sind.

15. Dentales Röntgengerät, **gekennzeichnet durch** eine Anordnung nach einem oder mehreren der vorhergehenden Ansprüche.

16. Verfahren zur Positionierung des Strahlers und/oder Strahlenempfängers eines dentalen Röntgengerätes unter Verwendung einer vorhandenen digitalisierten dentalen Röntgenaufnahme und der digitalisierten dentalen Röntgenaufnahme zuordenbaren Röntgengerätinformationen,
- bei dem mindestens eine digitalisierte dentale Röntgenaufnahme geladen und dargestellt wird,
- bei dem anhand der digitalisierten dentalen Röntgenaufnahme die Koordinaten von Bereichen bestimmt werden, die in einer weiteren Röntgenaufnahme abgebildet werden sollen,
- bei dem Röntgengerätinformationen geladen werden,
- bei dem auf der Grundlage der digitalisierten Röntgenaufnahme, den dazu vorliegenden Röntgengerätinformationen und des ausgewählten Bereichs Berechnungen durchführt werden, um Steuerungsdaten zu ermitteln, die das dentale Röntgengerät so steuern, dass der ausgewählte Bereich in einer dentalen Röntgenaufnahme abbildbar ist.

17. Verfahren nach dem vorhergehenden Anspruch, **dadurch gekennzeichnet, dass** die digitalisierte Röntgenaufnahme eine individuelle Aufnahme des Patienten ist.

18. Verfahren nach einem oder mehreren der vorhergehenden Ansprüche, **dadurch gekennzeichnet, dass** vor dem dritten Schritt eine Auswahl der Aufnahmeart des Röntgengeräts erfolgt.

19. Verfahren nach einem oder mehreren der vorhergehenden Ansprüche, **dadurch gekennzeichnet, dass** die Steuerungsdaten Mittel zur Positionierung des Patienten in bezug auf das Röntgengerät steuern.

20. Verfahren nach dem vorhergehenden Anspruch, **dadurch gekennzeichnet, dass** die Informationen des Röntgengerätes Koordinaten der Bahnkurve sind, die in Relation zu der digitalisierten Röntgenaufnahme abgelegt sind, und durch den ausgewählten Bereich ein Segment der Bahnkurve berechnet wird.

21. Verfahren nach einem oder mehreren der vorhergehenden Ansprüche, **dadurch gekennzeichnet, dass** Strom- und/oder Spannungsparameter in Relation zu der digitalisierten Röntgenaufnahme abgelegt sind.

22. Verfahren nach einem oder mehreren der vorhergehenden Ansprüche, **dadurch gekennzeichnet, dass** bei der Bestimmung der Steuerungsdaten die Untersuchungsart und/oder das Diagnoseziel des Patienten in die Berechnung einfließen.

23. Verfahren nach einem oder mehreren der vorhergehenden Ansprüche, **dadurch gekennzeichnet, dass** Patientendaten, wie Größe, Gewicht, Typ, Rasse, Alter, Kieferform und/oder bisherige Behandlungen bei der Bestimmung der Steuerungsdaten in die Berechnung einfließen.

24. Verfahren nach einem oder mehreren der vorhergehenden Ansprüche, **dadurch gekennzeichnet, dass** Bereiche, insbesondere Zähne, durch Mustererkennungsalgorithmen automatisch erkannt werden.

25. Verfahren nach einem oder mehreren der vorhergehenden Ansprüche, **dadurch gekennzeichnet, dass** die Bereiche manuell bestimmbar sind.

26. Verfahren nach einem oder mehreren der vorhergehenden Ansprüche, **dadurch gekennzeichnet, dass** statistische und/oder stochastische Verknüpfungen der einzelnen Parameter vorgenommen werden.

27. Verfahren nach einem oder mehreren der vorhergehenden Ansprüche, **dadurch gekennzeichnet, dass** Aufnahmeserien mit unterschiedlicher Positionierung ausgehend von der ausgewählten Position erstellt werden.

## Claims

1. A system for positioning dental X-ray apparatus, comprising
- an input and output device for interactive control,
- a storage area, in which at least one digitized dental X-ray image and information concerning the X-ray apparatus assignable to the digitized dental X-ray image are stored,
- a computer interface, via which information can be interchanged with the X-ray apparatus, a processing unit which effects calculations based on a selected area, in order to ascertain control data for the dental X-ray apparatus,
- wherein the dental X-ray apparatus is controllable by said control data such that the selected area is covered when a dental X-ray image is made,
- means for selecting areas in the digitized dental X-ray image are available, and in that the processing unit calculate the control data for the dental X-ray apparatus, based on the selected area of
- the digitized dental X-ray image, the relevant information concerning the X-ray apparatus.

2. A system as defined in the previous claim, **characterized in that** the digitized X-ray image is an individual image of the patient.

3. A system as defined in the previous claim and comprising X-ray apparatus suitable for various types of image, **characterized in that** means for selecting the type of image are provided.

4. A system as defined in any one or more of the previous claims and comprising means for positioning a patient relatively to the X-ray apparatus, **characterized in that** the control data control said means for positioning the patient.

5. A system as defined in any one or more of the previous claims, **characterized in that** the information concerning the X-ray apparatus consists of the coordinates of the trajectory which have been saved in relation to the digitized X-ray image.

6. A system as defined in any one or more of the previous claims, **characterized in that** current and/or voltage parameters are saved in relation to the digitized X-ray image.

7. A system as defined in any one or more of the previous claims, **characterized in that** information concerning the gray tones in the representation of the image are saved in relation to the digital X-ray image.

8. A system as defined in any one or more of the previous claims, **characterized in that** computation for determining said control data takes into account the type of image.

9. A system as defined in any one or more of the previous claims, **characterized in that** computation for determining said control data takes into account the purpose of diagnosis.

10. A system as defined in any one or more of the previous claims, **characterized in that** patient-dependent data, such as size, weight, type, race, age, jaw shape, and/or previous treatments are taken into account when determining said control data.

11. A system as defined in any one or more of the previous claims, **characterized by** means for automatically recognizing areas, particularly teeth, by pattern recognition algorithms.

12. A system as defined in any one or more of the previous claims, **characterized in that** the selecting means are designed such that areas can be selected manually.

13. A system as defined in any one or more of the previous claims, **characterized in that** statistical and/or stochastic linkings of the individual parameters are carried out.

14. A system as defined in any one or more of the previous claims, **characterized in that** means are provided for making series of radiograms at different positions starting from the selected position.

15. Dental X-ray apparatus, **characterized by** a system as defined in any one or more of the previous claims.

16. A method of positioning the emitter and/or detector of dental X-ray apparatus using an existing digitized dental X-ray image and information concerning the X-ray apparatus and assignable to the digitized dental X-ray image, wherein
- at least one digitized dental X-ray image is loaded and displayed,
- coordinates of those areas are determined, with reference to the digitized dental X-ray image, which are to be depicted in another X-ray image,
- information concerning the X-ray apparatus is loaded,
- computation is carried out on the basis of the digitized X-ray image, the relevant information concerning the X-ray apparatus, and the selected area, in order to ascertain control data which controls the dental X-ray apparatus such that the selected area can be depicted in a dental X-ray image.

17. A method as defined in the previous claim, **characterized in that** the digitized X-ray image is an individual image of the patient.

18. A method as defined in any one or more of the previous claims, **characterized in that** the type of image to be made by the X-ray apparatus is selected prior to the third step.

19. A method as defined in any one or more of the previous claims, **characterized in that** the control data control means for positioning the patient relatively to the X-ray apparatus.

20. A method as defined in the previous claim, **characterized in that** the information concerning the X-ray apparatus comprises coordinates of the trajectory which have been saved in relation to the digitized X-ray image, and a segment of the trajectory is calculated on the basis of the selected area.

21. A method as defined in any one or more of the previous claims, **characterized in that** current and/or voltage parameters are saved in relation to the digitized X-ray image.

22. A method as defined in any one or more of the previous claims, **characterized in that** computation for determination of the control data takes into account the type of examination and/or the purpose of diagnosis of the patient.

23. A method as defined in any one or more of the previous claims, **characterized in that** patient-dependent data, such as size, weight, type, race, age, jaw shape, and/or previous treatments, are taken into account when computing the control data.

24. A method as defined in any one or more of the previous claims, **characterized in that** areas, particularly teeth, are automatically recognized by pattern recognition algorithms.

25. A method as defined in any one or more of the previous claims, **characterized in that** the areas can be determined manually.

26. A method as defined in any one or more of the previous claims, **characterized in that** statistical and/or stochastic linkings of the individual parameters are carried out.

27. A method as defined in any one or more of the previous claims, **characterized in that** series of radiograms are made at different positions starting from the selected position.

## Revendications

1. Agencement pour le positionnement d'un appareil radiographique dentaire doté :
- d'un appareil entrée/sortie pour la commande interactive,
- d'une plage de mémoire dans laquelle sont stockées au moins une radiographie dentaire numérisée et les informations de l'appareil radiographique pouvant être attribuées à la radiographie dentaire numérisée,
- d'une interface via laquelle des informations sont échangées avec l'appareil radiographique,
- d'une unité de traitement qui réalise des calculs sur la base d'une zone sélectionnée pour déterminer des données de commande pour l'appareil radiographique dentaire, l'appareil radiographique dentaire pouvant être commandé au moyen des données de commande de manière que la zone sélectionnée soit couverte par la prise d'une radiographie numérisée,
**caractérisé en ce que** des moyens sont disponibles pour sélectionner des zones sur la radiographie dentaire numérisée et **en ce que** l'unité de traitement pour le calcul des données de commande pour l'appareil radiographique les calcule sur la base de la zone sélectionnée de la radiographie dentaire numérisée et des informations de l'appareil radiographique disponibles pour celle-ci.

2. Agencement selon l'une quelconque des revendications précédentes, **caractérisé en ce que** la radiographie numérisée est une prise de vue individuelle du patient.

3. Agencement selon l'une quelconque des revendications précédentes doté d'un appareil radiographique approprié pour plusieurs types de prise de vue, **caractérisé en ce que** des moyens sont prévus pour sélectionner le type de prise de vue.

4. Agencement selon l'une ou plusieurs des revendications précédentes doté de moyens de positionnement d'un patient par rapport à l'appareil radiographique, **caractérisé en ce que** les données de commande commandent les moyens de positionnement du patient.

5. Agencement selon l'une ou plusieurs des revendications précédentes, **caractérisé en ce que** les informations de l'appareil radiographique sont des coordonnées de la portion de trajectoire enregistrées en association avec la radiographie numérisée.

6. Agencement selon l'une ou plusieurs des revendications précédentes, **caractérisé en ce que** des paramètres de courant et/ou de tension sont enregistrés en association avec la radiographie numérisée.

7. Agencement selon l'une ou plusieurs des revendications précédentes, **caractérisé en ce que** des informations sur les valeurs de gris de la représentation de la reproduction sont enregistrées en association avec la radiographie numérique.

8. Agencement selon l'une ou plusieurs des revendications précédentes, **caractérisé en ce que** le type de prise de vue est intégré dans le calcul lors de la détermination des données de commande.

9. Agencement selon l'une ou plusieurs des revendications précédentes, **caractérisé en ce que** l'objectif du diagnostic est intégré dans le calcul lors de la détermination des données de commande.

10. Agencement selon l'une ou plusieurs des revendications précédentes, **caractérisé en ce que** les données du patient telles que sa taille, son poids, son sexe, sa race, son âge, la forme de sa mâchoire et/ou ses traitements préalables sont intégrés dans le calcul lors de la détermination des données de commande.

11. Agencement selon l'une ou plusieurs des revendications précédentes, **caractérisé par** des moyens qui détectent automatiquement des zones, notamment les dents, par des algorithmes de détection types.

12. Agencement selon l'une ou plusieurs des revendications précédentes, **caractérisé en ce que** les moyens de sélection sont conçus de manière à ce que des zones puissent être sélectionnées manuellement.

13. Agencement selon l'une ou plusieurs des revendications précédentes, **caractérisé en ce que** des associations statistiques et/ou stochastiques des différents paramètres sont réalisées.

14. Agencement selon l'une ou plusieurs des revendications précédentes, **caractérisé en ce que** des moyens de création de séries de radiographie avec différents positionnements sont prévus en partant de la position sélectionnée.

15. Appareil radiographique dentaire **caractérisé par** un agencement selon l'une ou plusieurs des revendications précédentes.

16. Procédé de positionnement de la source de rayonnement et/ou du récepteur des rayons d'un appareil radiographique dentaire utilisant une radiographie dentaire numérisée existante et les informations de l'appareil radiographique pouvant être attribuées à la radiographie dentaire numérisée,
- dans lequel au moins une radiographie dentaire numérisée est chargée et représentée,
- dans lequel la radiographie dentaire numérisée permet de déterminer les coordonnées des zones qui doivent être reproduites dans une autre radiographie,
- dans lequel des informations de l'appareil radiographique sont chargées,
- dans lequel des calculs sont réalisés sur la base de la radiographie numérisée, des informations de l'appareil radiographique existant par rapport à celle-ci et de la zone sélectionnée pour déterminer des données de commande qui commandent l'appareil radiographique dentaire de manière à ce que la zone sélectionnée puisse être représentée sur une radiographie dentaire.

17. Procédé selon la revendication précédente, **caractérisé en ce que** la radiographie numérisée est une prise de vue individuelle du patient.

18. Procédé selon une ou plusieurs des revendications précédentes, **caractérisé en ce qu'**une sélection du type de prise de vue de l'appareil radiographique a lieu avant la troisième étape.

19. Procédé selon une ou plusieurs des revendications précédentes, **caractérisé en ce que** les données de commande commandent des moyens de positionnement du patient par rapport à l'appareil radiographique.

20. Procédé selon une ou plusieurs des revendications précédentes, **caractérisé en ce que** les informations de la radiographie sont des coordonnées de la portion de trajectoire enregistrées en association avec la radiographie numérisée et **en ce qu'**un segment de la portion de trajectoire est calculé par la zone sélectionnée.

21. Procédé selon une ou plusieurs des revendications précédentes, **caractérisé en ce que** des paramètres de courant et/ou de tension sont enregistrés en association avec la radiographie numérisée.

22. Procédé selon une ou plusieurs des revendications précédentes, **caractérisé en ce que** lors de la détermination des données de commande le type d'examen et/ou l'objectif du diagnostic du patient sont intégrés dans le calcul.

23. Procédé selon une ou plusieurs des revendications précédentes, **caractérisé en ce que** les données du patient telles que sa taille, son poids, son sexe, sa race, son âge, la forme de sa mâchoire et/ou ses traitements préalables sont intégrés dans le calcul lors de la détermination des données de commande.

24. Procédé selon une ou plusieurs des revendications précédentes, **caractérisé en ce que** des zones, notamment les dents, sont détectées automatiquement par des algorithmes de détection types.

25. Procédé selon une ou plusieurs des revendications précédentes, **caractérisé en ce que** les zones peuvent être déterminées manuellement.

26. Procédé selon une ou plusieurs des revendications précédentes, **caractérisé en ce que** des associations statistiques et/ou stochastiques des différents paramètres sont réalisées.

27. Procédé selon une ou plusieurs des revendications précédentes, **caractérisé en ce que** des séries de prise de vue sont créées avec différents positionnements en partant de la position sélectionnée.
